(19) Europäisches Patentamt
European Patent Office
Office européen des brevets

(11) EP 0 972 761 B1

(12) **EUROPEAN PATENT SPECIFICATION**

(45) Date of publication and mention of the grant of the patent:
**12.12.2001 Bulletin 2001/50**

(51) Int Cl.⁷: **C07C 381/12**

(21) Application number: **99305552.4**

(22) Date of filing: **13.07.1999**

(54) **Sulfonium salt and its manufacturing method**

Sulfoniumsalze und Verfahren zu ihrer Herstellung

Sels de sulfonium et procédé pour leur préparation

(84) Designated Contracting States:
**AT BE CH CY DE DK ES FI FR GB GR IE IT LI LU MC NL PT SE**

(30) Priority: **16.07.1998 KR 2883398**
**03.09.1998 EP 98307103**

(43) Date of publication of application:
**19.01.2000 Bulletin 2000/03**

(73) Proprietor: **Korea Kumho Petrochemical Co. Ltd.**
**Chongno-ku, Seoul 110-110 (KR)**

(72) Inventors:
- **Park, Joo-Hyeon**
**Yousung-ku, Taejeon-city (KR)**
- **Seo, Dong-Chul**
**Yousung-ku, Taejeon-city (KR)**
- **Park, Sun-Ju**
**Yousung-ku, Taejeon-city (KR)**
- **Kim, Seong-Ju**
**Yousung-ku, Taejeon-city (KR)**

(74) Representative: **Nash, David Allan et al**
**Haseltine Lake & Co.,**
**Imperial House, 15-19 Kingsway**
**London WC2B 6UD (GB)**

(56) References cited:
**US-A- 5 679 496**

- **R.D. MILLER ET AL: J. ORG. CHEM., vol. 53, no. 23, 1988, pages 5571-5573, XP002117748**
- **D.N. KEVILL ET AL: J. ORG. CHEM., vol. 56, no. 10, 1991, pages 3454-3457, XP002117749**
- **DATABASE CHEMABS [Online] CHEMICAL ABSTRACTS SERVICE, COLUMBUS, OHIO, US STN, CAPLUS, accession no. 1994:232080, XP002117756 & JP 05 232705 A (NIPPON TELEGRAPH & TELEPHONE)**
- **S.-J. KIM ET AL: PROC. SPIE-INT. SOC. OPT. ENG. (ADVANCES IN RESIST TECHNOLOGY AND PROCESSING XIV), vol. 3049, 1997, pages 430-436, XP002103824**
- **V.G. NENAJDENKO ET AL: J. ORG. CHEM., vol. 62, no. 8, - 1997 pages 2483-2486, XP002117750**
- **F.D. SAEVA ET AL: J. AM. CHEM. SOC., vol. 111, no. 4, 1989, pages 1328-1330, XP002117751**
- **ANONYMOUS: "Highly soluble, thermally stable photoacid initiators" RESEARCH DISCLOSURE, no. 337, May 1992 (1992-05), page 332 XP000309797**
- **DATABASE CHEMABS [Online] CHEMICAL ABSTRACTS SERVICE, COLUMBUS, OHIO, US STN, CAPLUS, accession no. 1996:262070, XP002117757 -& CHEMICAL ABSTRACTS, vol. 124, no. 22, 27 May 1996 (1996-05-27) Columbus, Ohio, US; abstract no. 302584b, XP002117753 & JP 08 027094 A (JAPAN SYNTHETIC RUBBER CO LTD)**
- **DATABASE CHEMABS [Online] CHEMICAL ABSTRACTS SERVICE, COLUMBUS, OHIO, US STN, CAPLUS, accession no. 1992:460751, XP002117758 & JP 04 042158 A (HITACHI LTD ET AL)**
- **DATABASE CHEMABS [Online] CHEMICAL ABSTRACTS SERVICE, COLUMBUS, OHIO, US STN, CAPLUS, accession no. 1997:154980, XP002117759 -& CHEMICAL ABSTRACTS, vol. 126, no. 13, 25 March 1997 (1997-03-25) Columbus, Ohio, US; abstract no. 179054r, XP002117755 & JP 09 012537 A (SHINETSU CHEM IND CO)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 972 761 B1

- **T. UMEMOTO ET AL: J. AM. CHEM. SOC., vol. 115, no. 6, 1993, pages 2156-2164, XP002117752**

## Description

This invention relates to a method of manufacturing a sulfonium salt, represented by the following formula I, which is effectively used as a photoacid initiator or radical photoinitiator during polymerization and a photoacid generator leaving the protection groups of organic compounds.

**Formula I**

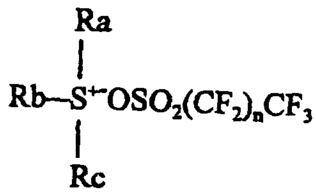

in which, Ra, Rb and Rc are independently alkyl group, aryl group, allyl group, aromatic compound or benzyl group, respectively; n is an integer of 0 to 20.

## Discussion of Related Art

In general, sulfonium salt is being effectively used as a photoacid initiator or radical photoinitiator during polymerization and a photoacid generator leaving the protection groups of organic compounds. Further, the sulfonium salt has been recently spotlighted as a photoacid generator of the chemically amplified photoresist, being employed in semiconductor materials.

According to the conventional method, it has been disclosed that the sulfonium salt is synthesized via two-step reaction in the presence of Grignard reagent (J. Am. Chem. Soc., 1990, 112, 6004-6015). Nevertheless, the conventional method recognized some disadvantages in that excess of Grignard reagent should be inevitably added in the two-step reaction and the full-scale industrial production has proven to be unavailable due to poor yield of final product.

Under the consideration of such a technological problem, further research has focused on the method of synthesizing the sulfonium salt via one-step reaction in the presence of trialkylsilyl triflate (J. Org. Chem, 1988, 53, 5571-5573). However, such method has failed to improve the poor yield related problem.

## SUMMARY OF THE INVENTION

An object of this invention is to provide a method of manufacturing sulfonium salt, wherein one-step reaction between sulfoxide compound and aromatic compound instead of Grignard reagent is performed in the presence of perfluoroalkanesulfonic anhydride, thus making it available to prepare a novel sulfonium salt with higher yield which cannot be achieved in the prior art and also to prepare even any conventional sulfonium salt having better yield.

To achieve the above objective, the sulfonium salt of this invention is characterized by the following formula 1.

**Formula I**

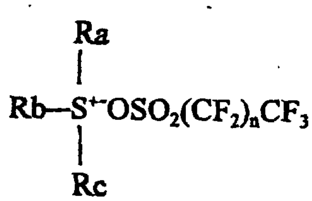

in which,

Ra, Rb and Rc are independently alkyl group, aryl group, allyl group,

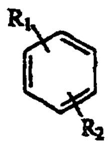
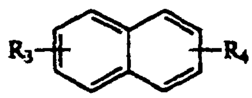
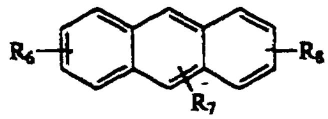
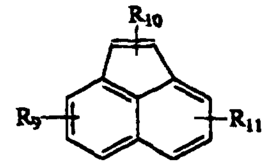
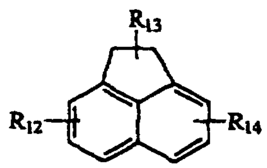
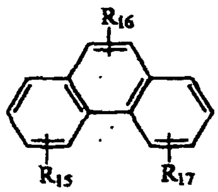
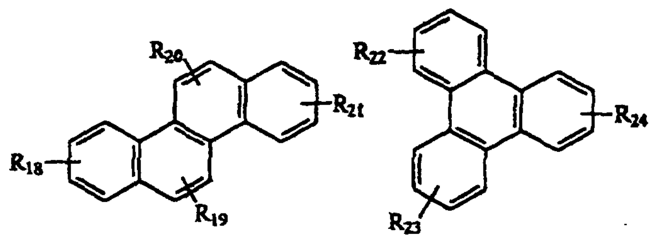
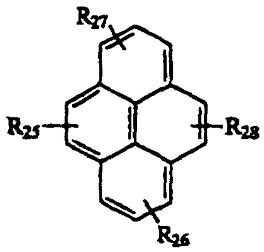
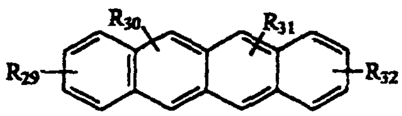
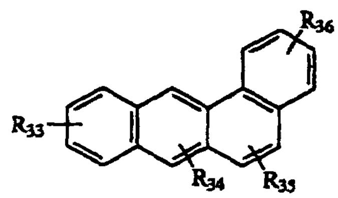
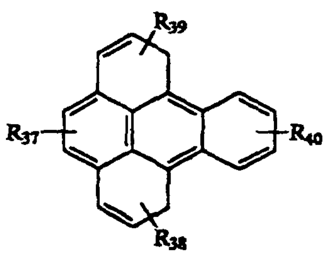
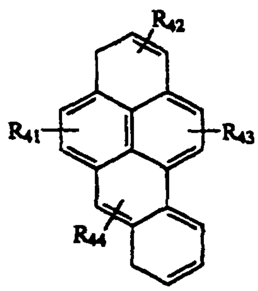
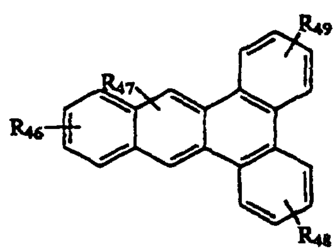

or benzyl group respectively;

$R_1$ to $R_{49}$ are independently hydrogen atom, alkyl group, vinyl group, allyl group, aryl group, benzyl group, hydroxy group, thiol group, halogen atom, ester group, aldehyde group, alkoxy group, thioalkoxy group, phenoxy goup, thiophenoxy group, or nitrile group;

n is an integer of 0 to 20.

The sulfonium salt represented by the formula I is manufactured via a reaction among sulfoxide compound expressed by the following formula II, an aromatic compound represented by the following formula III and perfluoroalkanesulfonic anhydride represented by the following formula IV.

**Formula II**

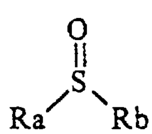

Where, Ra and Rb are the same as defined above.

**Formula III**

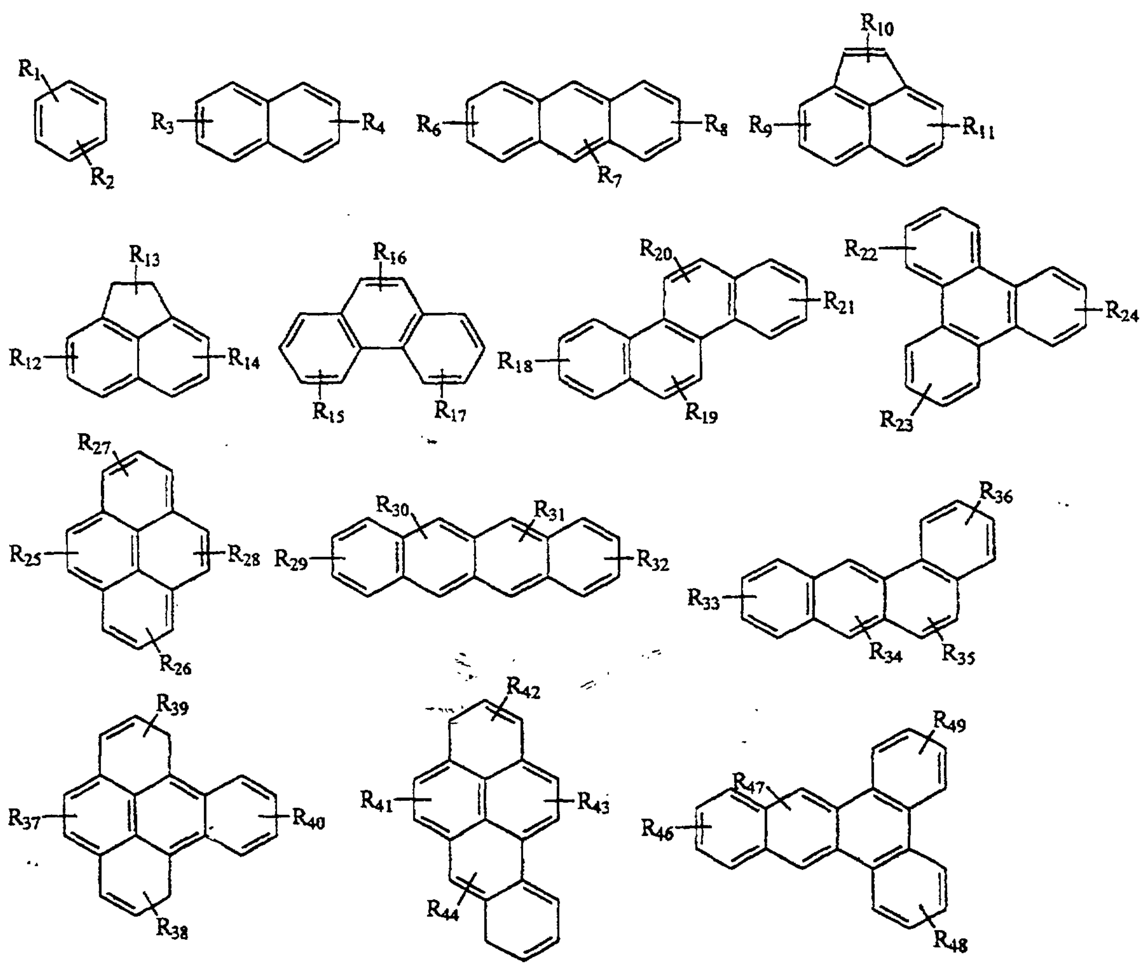

in which, $R_1$ to $R_{49}$ are the same as defined above.

**Formula IV**

$$[CF_3(CF_2)nSO_2]_2-O$$

in which, n is the same as defined above.

## DETAILED DESCRIPTION OF PREFERRED EMBODIMENT

This invention is explained in more detail as set forth hereunder.

The method of manufacturing the sulfonium salt according to this invention is based on one-step reaction in which both sulfoxide compound and aromatic compound dissolved in a solvent are stirred in the presence of perfluoroalkanesulfonic anhydride.

The method of manufacturing the sulfonium salt according to this invention is represented by the following reaction scheme.

Scheme 1

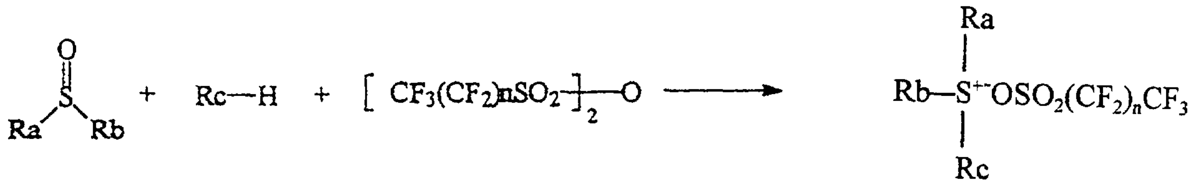

in which, Ra, Rb, Rc and n are the same as defined above.

As defined above, the sulfoxide compound of this invention, represented by the formula II, may include any one of common sulfoxide compounds selected from the group consisting of alkyl sulfoxide, aryl sulfoxide, alkylaryl sulfoxide, allyl sulfoxide, alkylallyl sulfoxide, and arylallyl sulfoxide.

Among the above compounds, however, the reactability of alkyl sulfoxide is slightly lower than that of aryl sulfoxide. Further, the sulfonium compound with higher yield may be obtained by those compounds regardless of functional groups present in sulfoxide compounds; examples of the functional groups include ester group, ether group, thioester group, sulfanyl group, alcohol group, amide group, ketone group, aldehyde group, and halogen group.

The aromatic compound represented by the formula III may contribute to higher yield of a desired product regardless of any functional groups. If there are any functional groups having excellent selective substitution power at the para-position, a lot of para-oriented products are obtained.

Meantime, if the reaction temperature is high, the selective substitution power at the para-position is slightly reduced. For example, when phenyl sulfoxide, toluene and trifluoromethanesulfonic anhydride are reacted at below 0°C, diphenyl(4-methylphenyl)sulfonium triflate having the selective substitution power at the para-position is only generated, while a small amount of diphenyl(2-methylphenyl)suifonium triflate having the selective substitution power at the ortho-position is generated at more than room temperature.

The amount of aromatic compound represented by the formula III is in the range of 1 to 100 equivalents per equivalent of sulfoxide compound represented by the formula II, preferably in the range of 1 to 1.5 equivalents.

Meantime, perfluoroalkanesulfonic anhydride may include all of the common compounds and among them, the typical compound is trifluoromethanesulfonic anhydride.

The amount of perfluoroalkanesulfonic anhydride is in the range of 1 to 5 equivalents per equivalent of sulfoxide compound represented by the formula U, preferably in the range of 1 to 1.5 equivalents.

During the process of manufacturing the sulfonium salt using those compounds, examples of reaction solvent include dichloromethane, chloroform, carbon tetrachloride, acetonitrile, ethyl acetate, ethyl ether, and dioxane; among them, haloalkane compounds are rather appropriate, even if unspecified.

According to this invention, the aromatic compound represented by the formula III may be employed as a reacting solvent as well as a reactant in consequence. However, as a case demands, the non-use of such aromatic compound as solvent does not raise any problem.

In addition, the manufacture of sulfonium salt is performed at -80 to 100°C; if the reaction in temperature is extremely high, the generation of byproducts may be responsible for poor yield. Occasionally, when the reaction temperature increases, a mixture of both para-oriented and ortho-oriented materials may be generated.

As for a method of manufacturing the sulfonium salt according to this invention unlike the prior arts, the final product may be synthesized using alkyl sulfoxide; further, the sulfonium salt substituted by halogen or ester group may be freely prepared. It is nearly impossible to prepare the sulfonium salt in the conventional method using Grignard reagent.

When the patterning of pattern in a photoresist composition derived from the sulfonium salt of this invention together with some basic resin and additives in small amounts are examined, better photoresist pattern is observed than the conventional sulfonium salt. As a matter of course, the sulfonium salt of this invention is effectively used as a photoacid initiator or radical photoinitiator during polymerization and a catalyst leaving the protection groups of organic compounds.

This invention is explained in more detail based on the following Examples but is not limited by those Examples. The sulfonium salts prepared by the following Examples are shown in the following table 1.

**Table 1**

| Exam | Formula | m.p.,°C | Exam | Formula | m.p., °C |
|---|---|---|---|---|---|

| | | | | | |
|---|---|---|---|---|---|
| 2 | 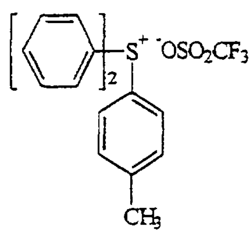 | 132-134 | 3 | 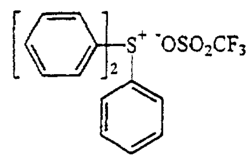 | 101-102 |
| 4 | 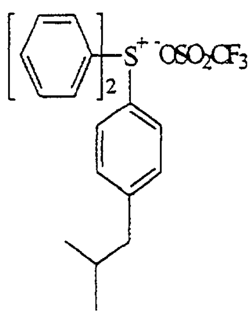 | 100-101 | 5 | 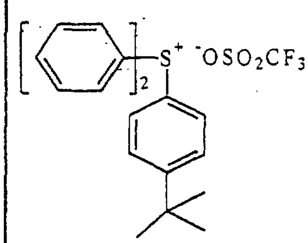 | 112-113 |
| 6 | 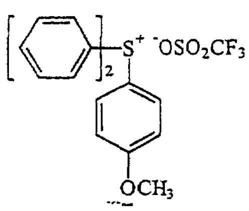 | 98-100 | 7 | 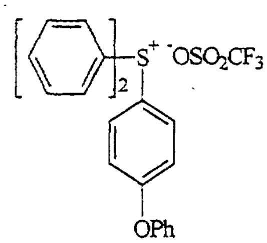 | 89-91 |
| 8 | 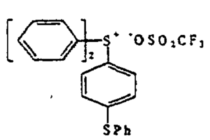 | 81-82 | 9 | 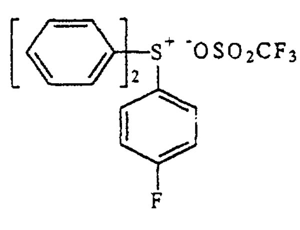 | 117-118 |

| | | | | | |
|---|---|---|---|---|---|
| 10 | 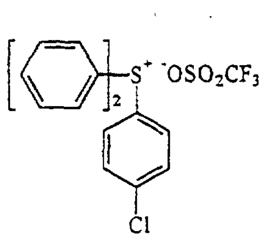 | 111-112 | 11 | 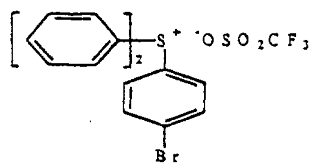 | 111-112 |
| 12 | 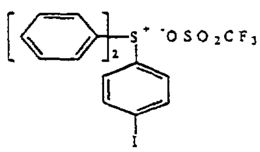 | 151-152 | 13 | 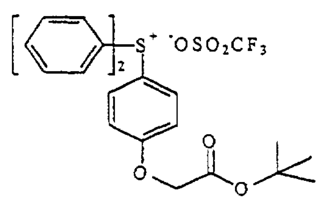 | 91-92 |
| 14 | 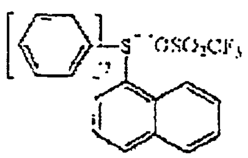 | 132-133 | 15 | 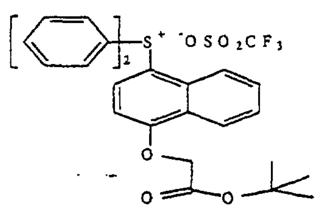 | 145-146 |

| | | | | | |
|---|---|---|---|---|---|
| 16 | 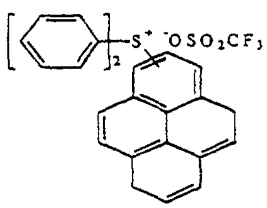 | | 17 | 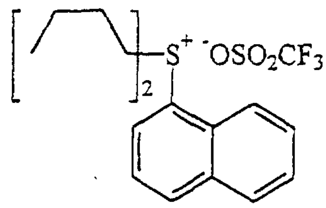 | 103-104 |
| 18 | 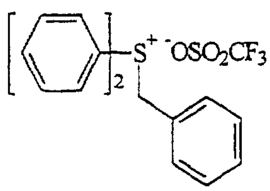 | 94-95 | 19 | 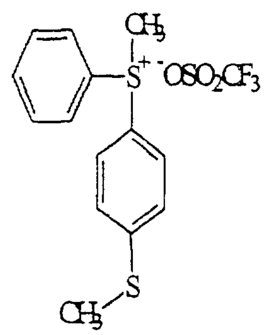 | 76-77 |
| 20 | 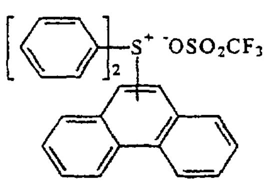 | | 21 | 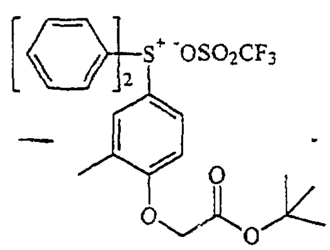 | |

| | | | | | |
|---|---|---|---|---|---|
| 22 | 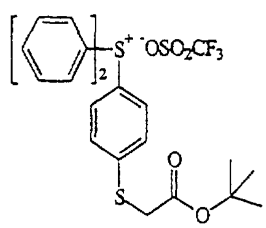 | | 23 | 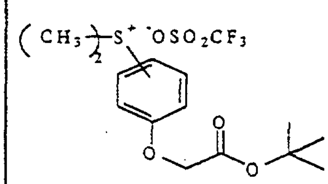 | |

**Example 1: Preparation of diphenyl(methyl phenyl)sulfonium triflate**

1g of phenyl sulfoxide dissolved in 50mL toluene was stirred at room temperature with a slow addition of 1.48g of triflic anhydride and further stirred for 1 hour.

Then, the sulfonium salt contained in the reacting mixture was extracted with distilled water and further, toluene used as a solvent and reactant was removed. The sulfonium salt, so extracted with distilled water, was re-extracted with dichloromethane into organic layer and then, the extraction solvent dichloromethane was removed under pressure. After the solvent was completely removed, an oil phase with larger viscosity was obtained. The oil phase, so formed, was completely dissolved in dichloromethane and with a slow addition of ether, a white precipitate was obtained.

As a final step, the white precipitate was filtered ,and dried by vaccum oven to obtain 2.06g of the sulfonium salt in a white solid (yield: 96%).

As a result of analyzing the mixture, so formed, on $^{1}$H-NMR and $C^{13}$-NMR, it was understood that the mixture did contain both diphenyl(2-methyl phenyl)sulfonium triflate and diphenyl(4-methylphenyl)sulfonium triflate in a ratio of 18:82.

**Example 2: Preparation of diphenyl(4-methyl phenyl)sulfonium triflate**

Both 1g of phenyl sulfoxide and 0.51g of toluene dissolved in 50mL dichloromethane was stirred and then, 1.48g of triflic anhydride was slowly added to the mixture. The reacting mixture was stirred at the same temperature for 30 minutes, and the reaction temperature was slowly increased up to room temperature. Then, the reacting mixture was washed with distilled water. After the complete removal of organic solvent contained in the washed organic layer under vacuum drying, an oil phase with larger viscosity was obtained. The oil phase, so formed, was completely dissolved in dichloromethane and with a slow addition of ether, a white precipitate was obtained. The precipitate was filtered ,and dried by vaccum oven to obtain 2.01g of diphenyl(4-methyl phenyl)sulfonium triflate (yield: 94%) in a white solid. The formula was shown in the table 1.

**Example 3: Preparation of triphenylsulfonium triflate**

In the procedure as described in Example 2, the sulfonium salt was prepared except that instead of using 0.51g of toluene, 0.43g of benzene was employed to obtain 1.89g of triphenylsulfonium trilflate (yield: 96%). The formula was shown in the table 1.

**Example 4: Preparation of diphenyl(4-isobutyl phenyl)sulfonium triflate**

In the procedure as described in Example 2, the sulfonium salt was prepared except that instead of using 0.51 g of toluene, 0.74g of isobutyl benzene was employed to obtain 2.13g of diphenyl(4-isobutyl phenyl)sulfonium triflate (yield: 91%). The formula was shown in the table 1.

**Example 5: Preparation of diphenyl(4-*tert*-butylphenyl)sulfonium triflate**

In the procedure as described in Example 2, the sulfonium salt was prepared except that instead of using 0.51g of toluene, 0.74g of *tert*-butylbenzene was employed to obtain 2.25g of diphenyl(4-tert-butylphenyl)sulfonium triflate (yield: 96%). The formula was shown in the table 1.

**Example 6: Preparation of diphenyl(4-methoxy phenyl)sulfonium triflate**

In the procedure as described in Example 2, the sulfonium salt was prepared except that instead of using 0.51g of toluene, 0.59g of anisole was employed to obtain 1.19g of diphenyl(4-methoxy phenyl)sulfonium triflate (yield: 90%). The formula was shown in the table 1.

**Example 7: Preparation of diphenyl(4-phenoxy phenyl)sulfonium triflate**

In the procedure as described in Example 2, the sulfonium salt was prepared except that instead of using 0.51g of toluene, 0.94g of phenyl ether was employed to obtain 2.37g of diphenyl(4-phenoxy phenyl)sulfonium triflate (yield: 94%). The formula was shown in the table 1.

**Example 8: Preparation of diphenyl(4-phenylsulfanyl phenyl)sulfonium triflate**

In the procedure as described in Example 2, the sulfonium salt was prepared except that instead of using 0.51g of toluene, 1.02g of phenylsulfide was employed to obtain 2.53g of diphenyl(4-phenylsulfanyl phenyl)sulfonium triflate (yield: 97%). The formula was shown in the table 1.

**Example 9: Preparation of diphenyl(4-fluorophenyl)sulfonium triflate**

In the procedure as described in Example 2, the sulfonium salt was prepared except that instead of using 0.51g of toluene, 0.53g of fluorobenzene was employed to obtain 1.85g of diphenyl(4-fluorophenyl)sulfonium )sulfonium triflate (yield: 86%). The formula was shown in the table 1.

**Example 10: Preparation of diphenyl(4-chlorophenyl)sulfonium triflate**

In the procedure as described in Example 2, the sulfonium salt was prepared except that instead of using 0.51g of toluene, 0.62g of chlorobenzene was employed to obtain 2.03g of diphenyl(4-chlorophenyl)sulfonium triflate (yield: 93%). The formula was shown in the table 1.

**Example 11: Preparation of diphenyl(4-bromophenyl)sulfoninm triflate**

In the procedure as described in Example 2, the sulfonium salt was prepared except that instead of using 0.51g of toluene, 0.86g of bromobenzene was employed to obtain 2.40g of diphenyl(4-bromophenyl)sulfonium triflate (yield: 98%). The formula was shown in the table 1.

**Example 12: Preparation of diphenyl(4-iodophenyl)sulfonium triflate**

In the procedure as described in Example 2, the sulfonium salt was prepared except that instead of using 0.51g of toluene, 1.12g of iodobenzene was employed to obtain 2.21g of diphenyl(4-iodophenyl)sulfonium triflate (yield: 82%). The formula was shown in the table 1.

**Example 13: Preparation of diphenyl(4-*tert*-butoxy carbomethoxy phenyl)sulfonium triflate**

In the procedure as described in Example 2, the sulfonium salt was prepared except that instead of using 0.51g of toluene, 1.15g of *tert*-butyl phenoxyacetate was employed to obtain 2.36g of diphenyl(4-*tert*-butoxycarbomethoxyphenyl)sulfonium triflate (yield: 87%). The formula was shown in the table 1.

**Example 14: Preparation of diphenyl(naphthalen-1-yl)sulfonium triflate**

In the procedure as described in Example 2, the sulfonium salt was prepared except that instead of using 0.51g of toluene, 0.70g of naphthalene was employed to obtain 2.29g of diphenyl(naphthalen-1-yl)sulfonium triflate

(yield: 99%). The formula was shown in the table 1.

**Example 15: Preparation of diphenyl(4-*tert*-butoxy carbomethoxy naphthalen-1- yl)sulfonium triflate**

In the procedure as described in Example 2, the sulfonium salt was prepared except that instead of using 0.51 g of toluene, 1.42g of tent-butyl 1-naphtyloxyacetate was employed to obtain 2.40g of diphenyl(4-*tert*-butoxy carbomethoxy naphthalen-1-yl)sulfonium triflate (yield: 81%). The formula was shown in the table 1.

**Example 16: Preparation of diphenyl(pyrenyl)sulfonium triflate**

In the procedure as described in Example 2, the sulfonium salt was prepared except that instead of using 0.51g of toluene, 1.11g of pyrene was employed to obtain 1.37g of diphenyl(pyrenyl)sulfonium triflate (yield: 51%). The formula was shown in the table 1.

**Example 17: Preparation of dibutyl(naphthalen-1-yl)sulfonium triflate**

In the procedure as described in Example 2, the sulfonium salt was prepared except that instead of using 0.51g of toluene and 1g of phenyl sulfoxide, 0.70g of naphthalene and 0.81 g of butyl sulfoxide, respectively, were employed to obtain 1.65g of dibutyl(naphthalen-1-yl)sulfonium triflate (yield: 78%). The formula was shown in the table 1.

**Example 18: Preparation of diphenyl(benzyl)sulfonium triflate**

In the procedure as described in Example 2, the sulfonium salt was prepared except that instead of using 0.51g of toluene and 1g of phenyl sulfoxide, 0.43 g of benzene and 1.08g of benzylphenyl sulfoxide, respectively, were employed to obtain 1.83g of dibutyl(benzyl)sulfonium triflate (yield: 86%). The formula was shown in the table 1.

**Example 19: Preparation of methyl(phenyl)(4-methylsulfanyl phenyl)sulfonium triflate**

In the procedure as described in Example 2, the sulfonium salt was prepared except that instead of using 0.51g of toluene and 1g of phenyl sulfoxide, 0.68g of thioanisole and 0.70g of methylphenyl sulfoxide, respectively, were employed to obtain 1.56g of methyl(phenyl)(4-methylsulfanyl phenyl)sulfonium triflate (yield: 79%). The formula was shown in the table 1.

**Example 20: Preparation of diphenyl(phenanthrenyl)sulfonium triflate**

In the procedure as described in Example 2, the sulfonium salt was prepared except that instead of using 0.51g of toluene, 0.98g of phenanthrene was employed to obtain 1.66g of diphenyl(phenanthrenyl)sulfonium triflate (yield: 65%). The formula was shown in the table 1.

**Example 21: Preparation of diphenyl(4-*tert*-butoxycarbomethoxy-3-methyl phenyl)sulfonium triflate**

In the procedure as described in Example 2, the sulfonium salt was prepared except that instead of using 0.51g of toluene, 1.22g of *tert*-butyl 2-tolyloxyacetate was employed to obtain 2.34g of diphenyl(4-*tert*-butoxycarbomethoxy-3-methylphenyl)sulfonium triflate in oil phase (yield: 84%). The formula was shown in the table 1.

**Example 22: Preparation of diphenyl(4-*tert*-butoxycarbothiomethoxy phenyl)sulfonium triflate**

in the procedure as described in Example 2, the sulfonium salt was prepared except that instead of using 0.51g of toluene, 1.23g of *tert*-butyl phenylsulfanylacetate was employed to obtain 2.29g of diphenyl(4-tent-butoxycarbothiomethoxy phenyl)sulfonium triflate in oil phase (yield: 82%). The formula was shown in the table 1.

**Example 23: Preparation of dimethyl(*tert*-butoxycarbomethoxy phenyl)sulfonium triflate**

In the procedure as described in Example 2, the sulfonium salt was prepared except that instead of using 0.51g of toluene and 1g of phenyl sulfoxide, 1.15g of *tert*-butyl phenoxyacetate and 0.39g of methyl sulfoxide was employed to obtain 1.48g of dimethyl(*tert*-butoxycarbomethoxyphenyl)sulfonium triflate in oil phase (yield: 72%). The formula was shown in the table 1.

**Comparative example 1: Preparation of tripheny sulfonium triflate**

I) Preparation of triphenyl sulfonium triflate

Phenyl magnesium bromide derived from 47g of bromobenzene and 7g of magnesium was prepared in the presence of absolute ether. After the removal of ether, a mixture of 50mL benzene and 100mL heptane was added to a reactor and then, the reaction temperature was increased up to 80°C. 12.1g of diphenyl sulfoxide was dissolved in 75mL benzene and then, this solution was slowly added to a solution containing phenyl magnesium bromide. Hence, the temperature of reacting solution was kept at 8°C and after the addition was completed, the reacting mixture was stirred at the same temperature for 3 hours and cooled to room temperature. With a slow addition of 200mL of 25% HBr aqueous solution, the aqueous solution layer was separated, while an organic layer was extracted two times using 30mL of 5% HBr aqueous solution. After the collection of the aqueous solution, so extracted, the solution was further extracted with 250mL dichloromethane. The dichloromethane layer, so extracted, was dried over anhydrous magnesium sulfate and then, the solvent was evaporated under vacuum drying. The remaining compound in oil phase was recrystallized using dichloromethane and ether, and dried to obtain 10.8g of triphenyl sulfonium triflate (yield: 52%).

II) Preparation of triphenyl sulfonium triflate

25.6g of triphenyl sulfonium triflate, so obtained from the above I), was dissolved in 200mL dichloromethane and added to 6.8mL triflic acid. The reacting solution was heated for 2 hours and after removal of HBr, a byproduct, was cooled to room temperature. The solution was washed with an aqueous solution of $NaHCO_3$ saturated with 50mL water. After the organic layer was separated, the solution was dried over anhydrous magnesium sulfate and then, dichloromethane was removed. Hence, a solid compound, so formed, was recrystallized with ethylacetate to obtain 25.6g of triphenyl sulfonium triflate (yield: 83%).

**Comparative example 2: Preparation of triphenyl sulfonium triflate**

2.0g of phenyl sulfoxide dissolved in 20mL dichloromethane was cooled to -78°C and then, 2.3mL trimethylsilyl triflate was slowly added to the mixture for more than 5 minutes. The reacting mixture was stirred at the same temperature for 10 minutes and with the gradual increase of reaction temperature, stirred at 0°C for 30 minutes. The solution was again cooled to -78°C and 10mL tetrahydrofuran solution of 2.0M phenyl magnesium bromide was slowly added to the reacting solution. The reacting mixture was stirred at the same temperature for 30 minutes and further stirred at 0°C for 30 minutes. The reaction was completed in 30mL of 3% triflic acid aqueous solution. The reactant was diluted with 200mL ether and extracted two times with 30mL of triflic acid aqueous solution. The aqueous solution, so extracted, was further extracted three times with 30mL chloroform. After collection of the organic layer, the solution was dried over anhydrous $Na_2SO_4$ and filtrated to remove the solvent. Thus, 1.9g of triphenyl sulfonium triflate (yield: 50%) was obtained as a white solid.

As described above in more detail, the sulfonium salt of this invention, so prepared via one-step reaction between sulfoxide compound and aromatic compound in the presence of perfluoroalkanesulfonic anhydride, has the following advantages in that a) by overcoming some shortcomings of the prior art to prepare the sulfonium salt via two steps using Grinard reagent, this invention may provide a novel sulfonium salt with higher yield which cannot be achieved in the prior art and also to prepare even any conventional sulfonium salt having better yield, and b) the sulfonium salt of this invention may be effectively used as a photoacid initiator or radical photoinitiator during polymerization and a photoacid generator leaving the protection groups of organic compounds, especially as an useful photoacid generator of the chemically amplified photoresist employed in semiconductor materials.

## Claims

1. A method of manufacturing the sulfonium salt represented by the formula I via reaction among sulfoxide compound represented by the following formula II, an aromatic compound represented by the following formula III and perfluoroalkanesulfonic anhydride represented by the following formula IV.

**Formula 1**

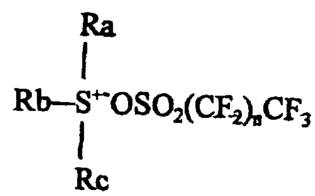

in which,

Ra, Rb and Rc are independently arkyl group, alyl group, allyl group,

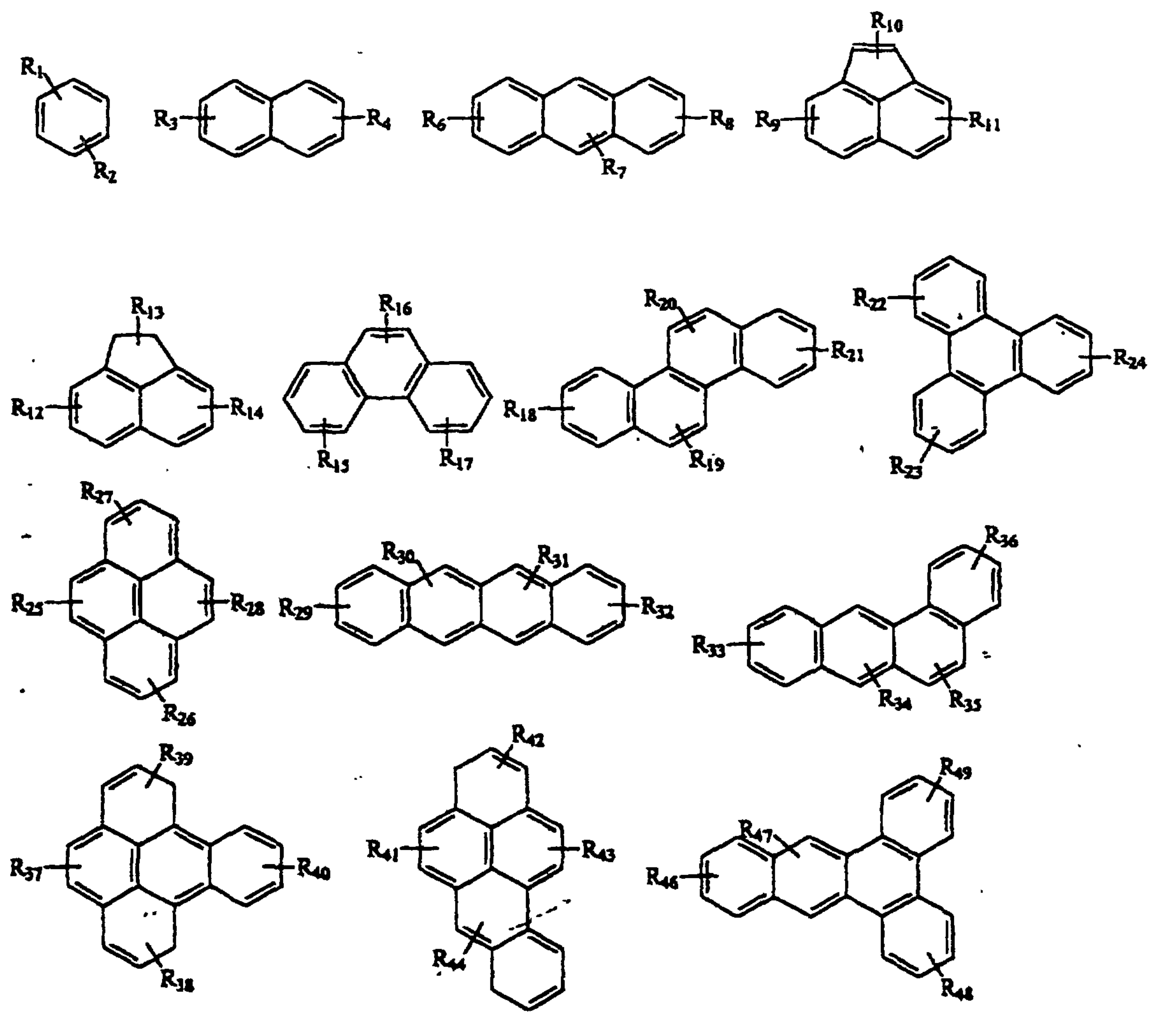

or benzyl group respectively;
$R_1$ to $R_{49}$ are independently hydrogen atom, alkyl group, vinyl group, allyl group, aryl group, benzyl group, hydroxy group, thiol group, halogen atom, ester group, aldehyde group, alkoxy group, thioalkoxy group, phenoxy group, thiophenoxy group, or nitrile group;
n is an integer of 0 to 20.

**Formula II**

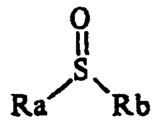

in which, Ra and Rb are the same as defined above.

**Formula III**

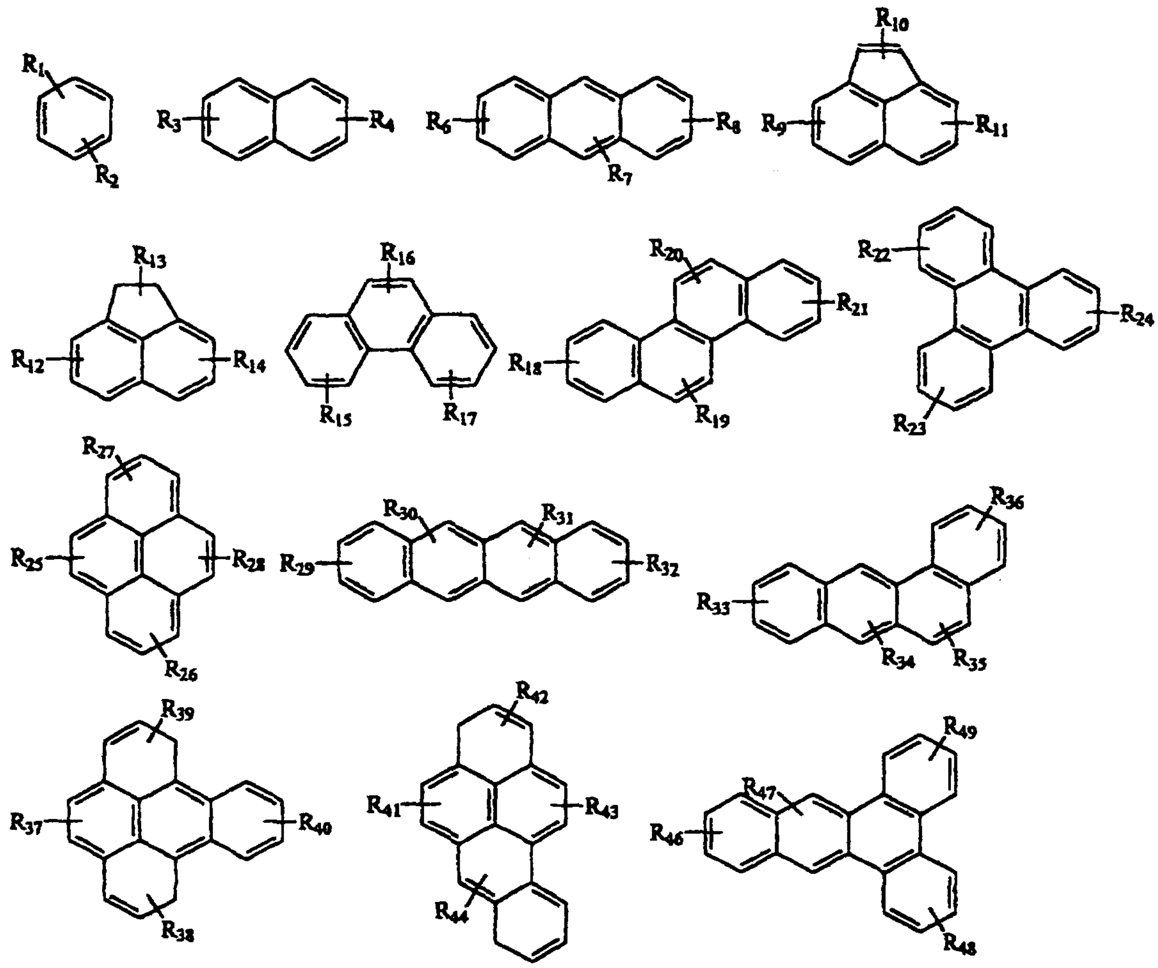

in which, $R_1$ to $R_{49}$ are the same as defined above.

**Formula IV**

$$[CF_3(CF_2)nSO_2]_2-O$$

in which, n is the same as defined above.

2. The method of claim 1, wherein the amount of an aromatic compound, represented by the formula III, is added in the range of 1 to 100 equivalents to a sulfoxide compound expressed by the formula II.

3. The method of claim 1, wherein the amount of perfluornalkanesulfonic anhydride, represented by the formula IV, is added in the range of 1 to 5 equivalents to a sulfoxide compound represented by the formula II.

4. The method of claim 1, wherein the reaction temperature is in the range of -80 to 100°C.

5. The method of claim 1, wherein the reaction solvent may be selected from the group consisting of haloalkane, haloalkene, ester, ether, alkane, alkene, aromatic compounds, amides and nitriles.

**Patentansprüche**

1. Verfahren zur Herstellung eines Sulfoniumsalzes der Formel I durch Umsetzen einer Sulfoxid-Verbindung gemäß nachstehender Formel II, einer Aromatenverbindung gemäß nachstehender Formel III sowie eines Perfluoralkansulfonsäureanhydrids gemäß nachstehender Formel IV, wobei gilt:

Formel I

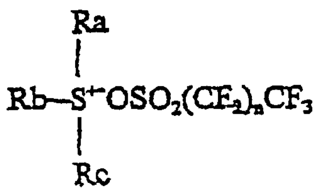

worin Ra, Rb und Rc unabhängig Alkyl-, Aryl-, und Allyl-Gruppen sind,

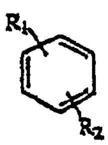
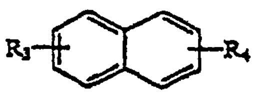
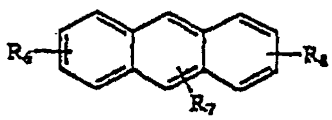
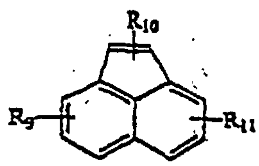

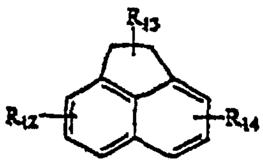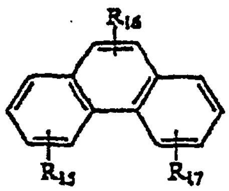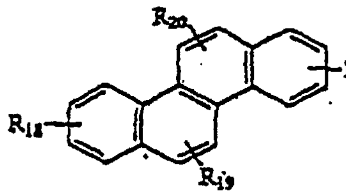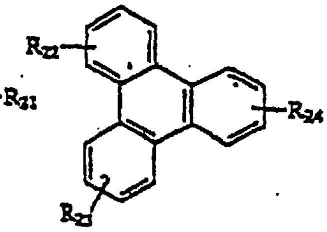

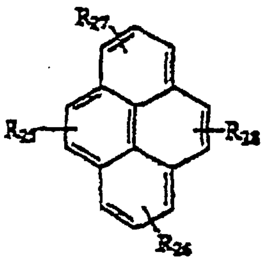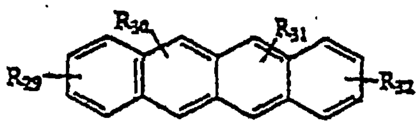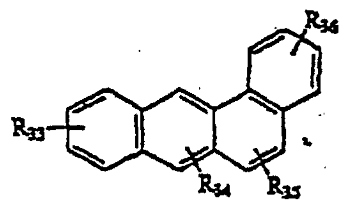

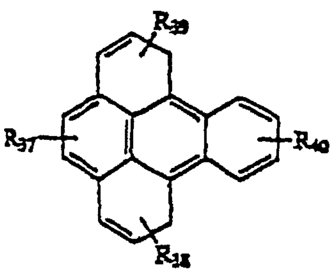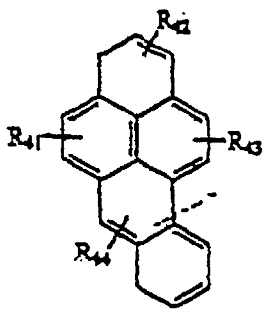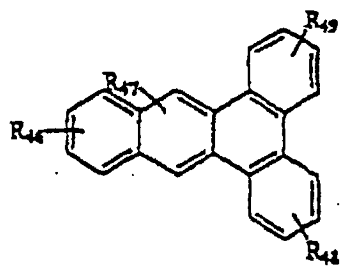

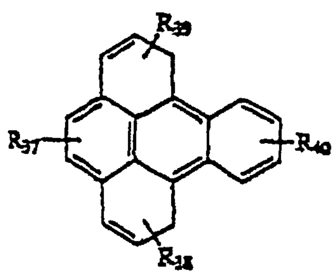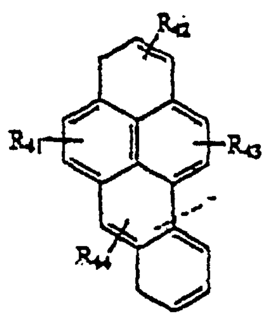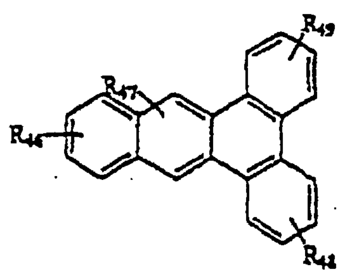

bzw. eine Benzylgruppe;

| | |
|---|---|
| $R_1$ bis $R_{49}$ | sind unabhängig Wasserstoffatom, Alkyl-Gruppe, Venyl-Gruppe, Allyl-Gruppe, Aryl-Gruppe, Benzyl-Gruppe, Hydroxy-Gruppe, Thiol-Grupppe, Halogen-Atom, Ester-Gruppe, Aldehyd-Gruppe, Alkoxy-Gruppe, Thioalkoxy-Gruppe, Phenoxy-Gruppe, Thiophenoxy-Gruppe oder Nitril-Gruppe; |
| n | eine ganze Zahl von 0 bis 20; |

Formel II

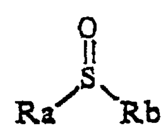

worin Ra und Rb wie oben definiert sind;

Formel III

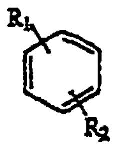
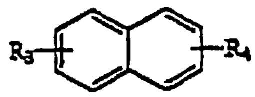
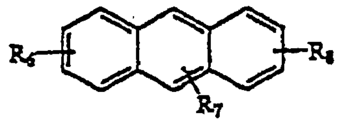
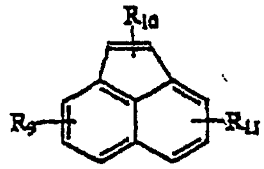
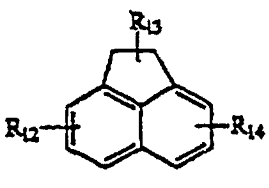
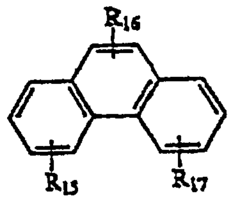
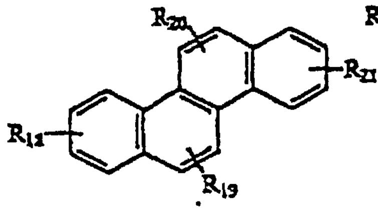
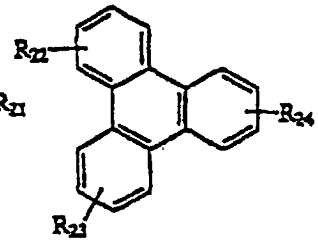
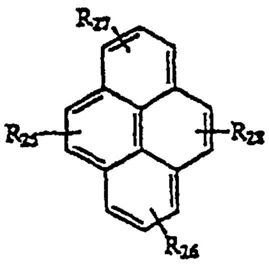
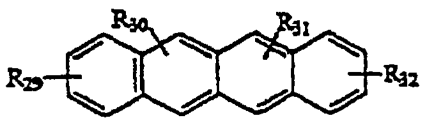
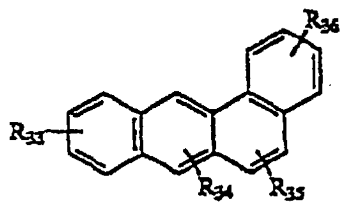
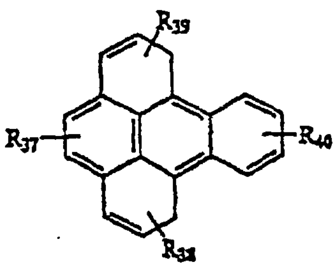
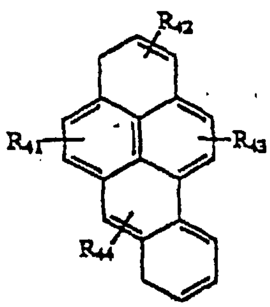
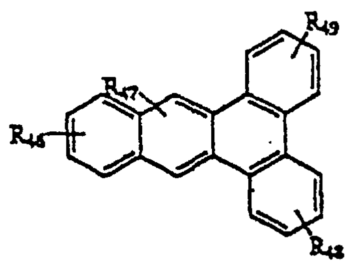

worin $R_1$ bis $R_{49}$ wie oben definiert sind;

Formel IV

$$[CF_3(CF_2)_nSO_2]_2O$$

worin n wie oben definiert ist.

**2.** Verfahren nach Anspruch 1, wobei die Menge an aromatischer Verbindung gemäß Formel III im Bereich von 1 bis 100 Äquivalenten zur Sulfoxid-Gruppe gemäß Formel II zugegeben wird.

**3.** Verfahren nach Anspruch 1, wobei die Menge Perfluoralkansulfonsäureanhidrid gemäß Formel IV im Bereich von 1 bis 5 Äquivalenten zur Sulfoxidverbindung gemäß Formel II zugegeben wird.

**4.** Verfahren nach Anspruch 1, wobei die Umsetzungstemperatur im Bereich von -80 bis 100° C ist.

**5.** Verfahren nach Anspruch 1, wobei das Reaktionslösungsmittel ausgewählt ist aus der Gruppe Haloalkane, Haloalkene, Ester, Ether, Alkane, Alkene, Aromatenverbindungen, Amide und Nitrile.

**Revendications**

**1.** Procédé de fabrication du sel de sulfonium représenté par la formule I par l'intermédiaire d'une réaction entre un composé sulfoxyde représenté par la formule II suivante, un composé aromatique représenté par la formule III suivante et un anhydride perfluoroalcanesulfonique représenté par la formule IV suivante :

Formule 1

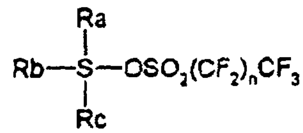

dans laquelle Ra, Rb et Rc sont indépendamment un groupe alkyle, un groupe aryle, un groupe allyle,

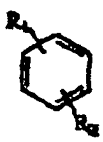
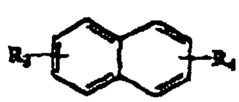
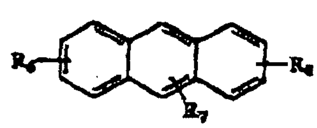
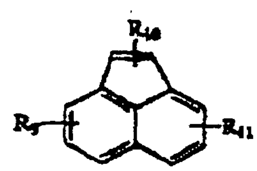
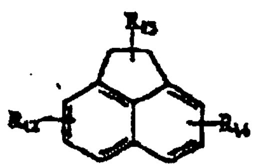
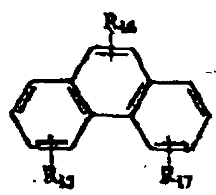
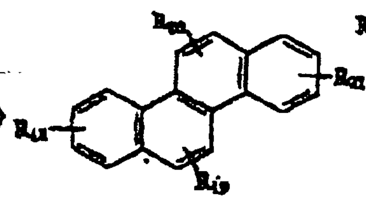
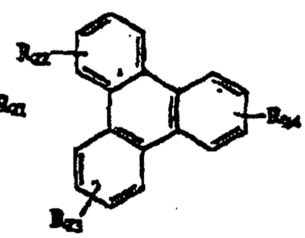
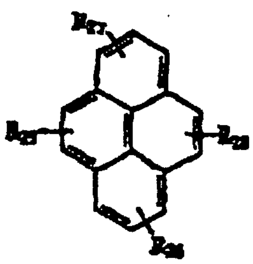
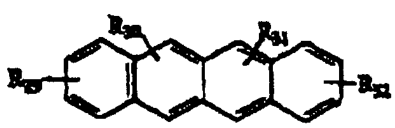
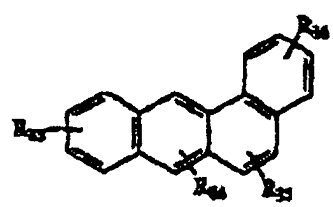
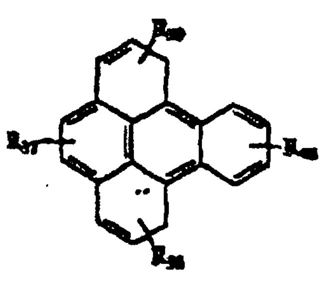
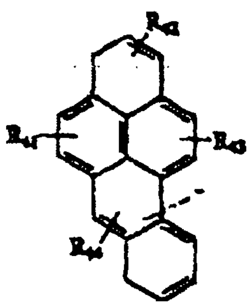
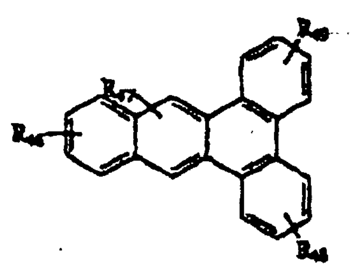

ou un groupe benzyle, respectivement ;
$R_1$ à $C_{49}$ sont indépendamment un atome d'hydrogène, un groupe alkyle, un groupe vinyle, un groupe allyle, un groupe aryle, un groupe benzyle, un groupe hydroxy, un groupe thiol, un atome d'halogène, un groupe ester, un groupe aldéhyde, un groupe alcoxy, un groupe thioalcoxy, un groupe phénoxy, un groupe thiophénoxy, ou un groupe nitrile ;

n est un entier de 0 à 20 ;

Formule II

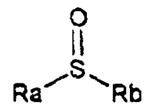

dans laquelle Ra et Rb sont tels que ceux definis ci-dessus ;

Formule III

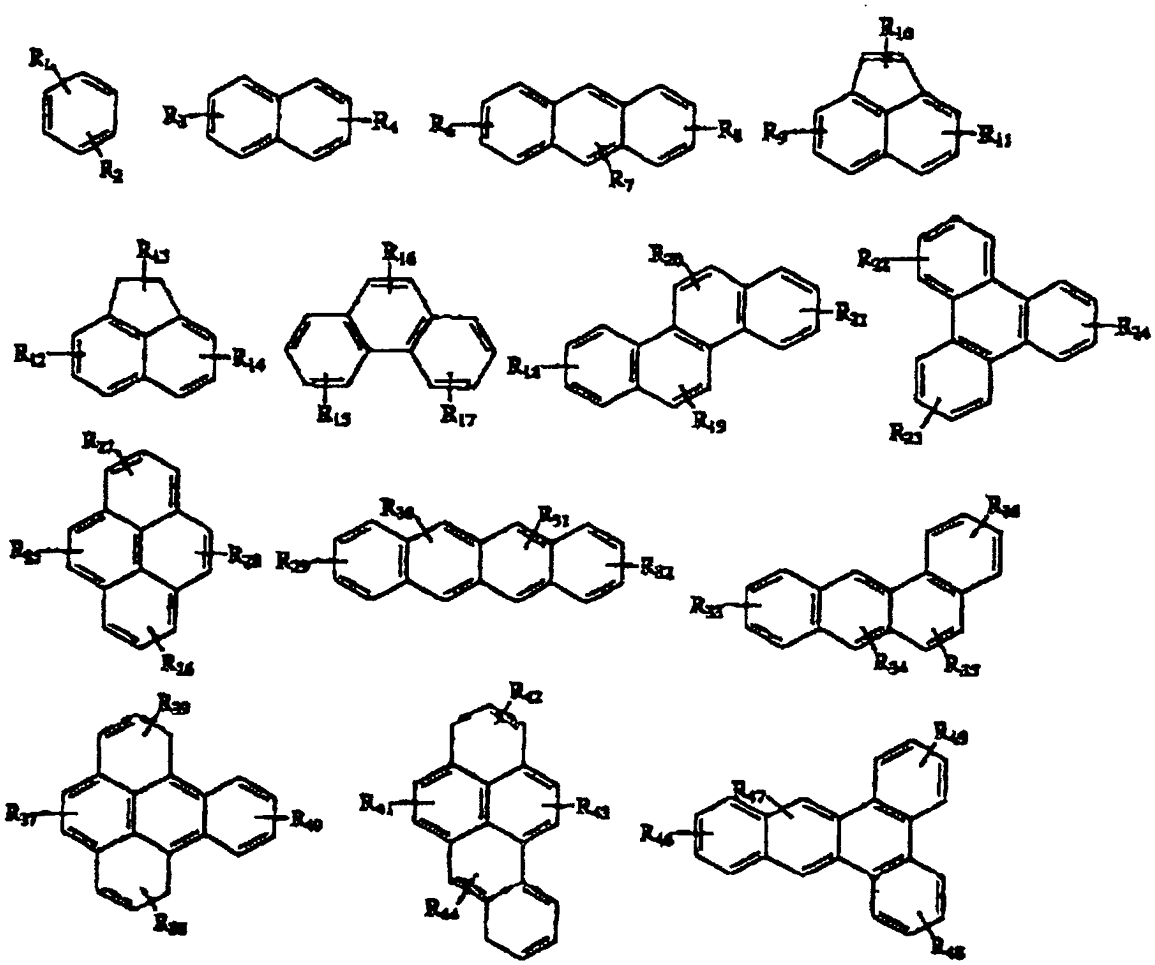

dans laquelle $R_1$ à $R_{49}$ sont tels que ceux définis ci-dessus ;

Formule IV

$$[CF_3(CF_2)_nSO_2)_2\text{-}O$$

dans laquelle n est tel que défini ci-dessus.

2. Procédé selon la revendication 1, dans lequel la quantité d'un composé aromatique, représenté par la formule III, est ajoutée à raison de 1 à 100 équivalents à un composé sulfoxyde exprimé par la formule II.

3. Procédé selor, la revendication 1, dans lequel la quantité d'anhydride perfluoroalcanesulfonique, représenté par

la formule IV, est ajoutée à raison de 1 à 5 équivalents à un composé sulfoxyde représenté par la formule II.

**4.** Procédé selon la revendication 1, dans lequel la température réactionnelle est située dans la plage allant de -80 à 100°C.

**5.** Procédé selon la revendication 1, dans lequel le solvant réactionnel peut être choisi dans l'ensemble constitué par un halogénoalcane, un halogénoalcène, un ester, un éther, un alcane, un alcène, les composés aromatiques, les amides et les nitriles.